# EUROPEAN PATENT APPLICATION

(11) **EP 2 127 694 A1**
(43) Date of publication of application: **02.12.2009**
(21) Application number: 08156922.0
(22) Date of filing: 26.05.2008
(51) Int. Cl.: A61N 1/20, A61N 1/32, A61B 5/053

(54) **Multi-electrode path for monitoring and electrical stimulation of wound healing**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

The present invention provides a device for electrical treatment of a skin wound comprising a flexible substrate adapted for attaching to the skin wound and the surrounding skin, a matrix of electrodes, means for determining the presence of the wound and/or its perimeter and means for applying a voltage between the wound and its surrounding. The present invention further provides a method for electrical treatment of a skin wound comprising the following steps: placing a matrix of electrodes attached to a flexible substrate on top of the skin wound; determining the presence of the wound and/or its perimeter; and charging at least some of the electrodes, wherein the electrodes at and/or close to the wound are charged oppositely with respect to the electrodes surrounding the wound.

## Description

### FIELD OF THE INVENTION

The present invention relates to a multi-electrode patch for electrical stimulation and monitoring of wound healing and to a method for electrical treatment of a skin wound.

### BACKGROUND OF THE INVENTION

Electrical stimulation (ES) of wound healing has been suggested as an adjunctive treatment for wound healing for many years. It involves the placement of electrodes in direct contact, or in close proximity, to a skin wound thereby creating an electrical current or voltage that passes through either the wound or peripheral tissue.

This form of accelerated wound healing has been the subject of many clinical trials over recent years which have culminated in health insurance companies (such as CIGNA) recognizing it as a genuine treatment and agreeing to compensate incurred treatment costs.

While the effects of ES have been known for several decades it is only recently that the mechanism behind the effect is being revealed. For example, a recent study by Zhao et al. (Nature 442, 457, 2006) showed that polarized phosphatidylinositol 3-kinase (PI3K) signaling steers the migration of human cells across a gradient of electrical potential. This process has been termed electrotaxis and has been observed by several other authors. In particular a publication from Huttenlocher (NEJM 356(3), 2007) clearly shows the movement of a cell under the influence of an electrical field. The movement is directed towards the cathode.

### SUMMARY OF THE INVENTION

However, there are several problems with prior art ES:

In the trials of ES that have so far been carried out one of the main problems is skin irritation and in extreme cases, skin burning. This is caused by too much current being locally drawn by the electrode section situated on top of the wound. If the wound (or an adjacent area of skin) has a low conductivity then the majority of current passes through this point, causing Joule heating and burning.

In existing devices, there is little lateral movement of cells due to the electrical current. The only movement is upwards from the stratum directly below the wound.
There is no monitoring of the wound and the healing process.

It has been shown that some waveforms are more effective for ES than others. These waveforms are limited to variations of the applied current in time. There are no possibilities to create any spatial variations, such as lateral electrical field patterns and currents directed towards the perimeter of the wound. Such waveforms could prove more efficient in accelerating healing.

There is therefore a need for an improved device for electrical stimulation of a skin wound and for an improved method of electrical treatment of skin wounds.

The present invention provides a device for electrical treatment of a skin wound comprising a flexible substrate adapted for attaching to the skin wound and the surrounding skin, a matrix of electrodes, means for determining the presence of the wound and/or its perimeter and means for applying a voltage between the wound and its surrounding.

The means for determining the presence of the skin wound may comprise one or more sensors for detecting the real-time state of the skin wound.

A "skin wound" according to the present invention may be any injury or lesion of the skin including irritations and inflammations. In particular, ES is preferably applicable in case of chronic wounds such as stage III or stage IV pressure ulcers, arterial diabetic ulcers and venous static ulcers.

According to a preferred embodiment, the means for applying a voltage is adapted to control the matrix of electrodes in such a way that the electrodes at and/or close to the wound are charged opposite with respect to the electrodes surrounding the wound or its perimeter at a predetermined distance. For example, the electrodes around and on top of the wound may be made negative and other electrodes further away may be made positive, or vice versa. Alternatively a counter electrode may be incorporated in the patch. In one preferred embodiment, the electrodes further away are chosen at a fixed distance from the perimeter of the wound, wherein the number of positive electrodes equals the number of negative electrodes. Naturally, a voltage between the wound and its surrounding may as well be achieved by different means. In general, the electrodes at and/or close to the wound and the electrodes surrounding the wound or its perimeter at a certain distance have to be set on different electric potentials. For instance, the electrodes around and on top of the wound may be grounded and other electrodes further away may be set on a positive potential.

More complex patterns of charge or potential may also be applied. It is, e.g., preferred that the means for applying a voltage is adapted to control the matrix of electrodes in such a way that the electric potential gradually increases or decreases from the electrodes at and/or close to the wound towards the electrodes surrounding the wound or its perimeter at a predetermined distance. This may be achieved by providing several rings of electrodes which are set on different electric potential. In another preferred embodiment, a gradient in voltage from the electrodes in the most outer regions of the matrix towards the electrodes situated near or on top of the wound is applied. Of course, the gradient may alternatively be directed from the center towards the periphery.

It is preferred that the device further comprises means for measuring the electric current caused by the applied voltage. Furthermore, the means for applying a voltage is adapted to control the matrix of electrodes in such a way that the measured current does not exceed a predetermined threshold. It is thus prevented that skin under the electrodes is irritated or even burned due to excessively high currents, which can cause Joule heating and consequently burning of skin.

The presence/location of the wound and/or its perimeter may be determined by measuring the impedance between each electrode and its neighboring electrodes. The voltage to be applied between at least some of the electrodes is then determined or calculated by an on-board or external control module in response to the location of the wound and/or its perimeter. Preferably the device is adapted for repeatedly determining the location and/or perimeter of the wound and readjusting the applied voltage pattern in predetermined time intervals. For instance, some wounds tend to heal from the perimeter towards the center. Accordingly, the largest voltage should be applied at the perimeter of such wounds. After some progress in healing the wound may be smaller and its perimeter may have migrated. Thus, the applied voltage pattern has to be re-adjusted in predetermined long-time intervals.

The means for applying a voltage may also be adapted to apply a varying voltage over short time intervals. For example, short DC current pulses may be applied, which travel towards the wound. Alternatively, AC electric fields may be used utilizing the effect of dielectrophoresis to stimulate the movement of cells towards the wound. Preferably, the voltage is between 1 V and 10 V and the frequency between 10 kHz and 100 kHz.

According to a preferred embodiment, the matrix of electrodes may be coated with a therapeutical agent. The therapeutical agent may comprise one or a combination of the following substances: growth factors, e.g. epidermal growth factors, polymer or lipid micells and/or vesicles suitable for drug delivery, corticosteroid-based drugs, anti-inflammatory proteins. If the electrodes, e.g., are coated with an epidermal growth factor and a gradient in voltage towards the wound is applied, the growth factor is "injected" into the wound. This should promote cell migration and accelerate healing.

Alternatively, the composition of vesicles, in particular of transport vesicles, can be tailored to encase therapeutic agents such as drugs or growth factors while the surface of the vesicle can be tailored to a specific charge. Hence such a "drug delivery" vesicle can be designed such that it responds to the electrical fields in a distinct manner from the natural environment (i.e. say inflammatory inducing cells). This can ensure the movement and localization of only said drug delivery vesicle, whilst leaving the surrounding tissue unperturbed.

Naturally, the type of therapeutical agent being suitable strongly depends on the type of wound. For instance, persisting inflammations (i.e. non-open wounds, with swelling, necrosis and inflammation) are often treated with inflammation suppression drugs. Such drugs act to suppress the localization and/or activation of immuno-cell at the sites of inflammation. Nonetheless, these drugs are not recommended for long term use, due to side effects and cellular adaptations. Much like cells can be stimulated electrically to migrate towards a source, one can also "repel" cells electrically and chemically. As described above, an electrically active patch can be applied over an inflamed area and the electric fields tuned to repulse inflammatory cells. This may provide a superior option to corticosteroid based drug therapies, since the negative effects of long term use would be ablated. In addition, as described above, the electric patch can also be used to create a locally defined gradient of chemo-repellant either via a charged drug, or anti-inflammatory protein, or via a complex drug delivery vehicle.

Preferably, the device according to the present invention comprises large area electronics on a polyimide release layer. However, the invention is not limited thereto and any type of flexible substrate suitable to be attached to skin may be used in combination with any matrix of electrodes.

In addition temperature sensors can be integrated into the electrically active patch so that the temperature of the wound can be measured. This is advantageous for detecting the presence of an infection as the skin temperature typically increases from 32C to 37C when a wound becomes infected. These sensors can also be formed in an array to allow a temperature map of the wound to be created. Most preferably the electrode geometry and material are chosen so that the electrode pad functions as both a resistive temperature sensor, a means for measuring impedance, and also for applying the voltage or current for healing.

In addition heaters can be integrated into the active patch so that the temperature profile over the wound can be controlled to influence the healing process.

Alternatively the mositure of the wound can also be measured via the electrically active patch and this measurement used to monitor the healing process.

Alternatively the active patch can use high frequency radiation, such as THz radiation, generated locally on the patch to monitor the healing process.

The present invention further provides a method for electrical treatment of a skin wound comprising the following steps: placing a matrix of electrodes attached to a flexible substrate on top of the skin wound; determining the presence of the wound and/or its perimeter; and applying a different potential to the electrodes at and/or close to the wound and to the electrodes surrounding the wound at a distance.

Preferably, the electric potential gradually increases or decreases from the electrodes at and/or close to the wound towards the electrodes surrounding the wound or its perimeter at a predetermined distance.

As already outlined with respect to the device, the steps of determining the location and/or perimeter of the wound and applying a potential to the electrodes are preferably repeated in predetermined time intervals in order to adjust the resulting applied voltage/current to the actual status of the wound and its progress in healing. The step of determining the location and/or perimeter of the wound may be performed by measuring the impedance between each electrode of the matrix and its neighboring electrodes. Since wounded skin has a different conductivity compared to healthy skin, the local impedance allows for determining the location of the wound.

The inventive method further comprises the step of measuring the current between the electrodes. The potential applied to the electrodes is controlled in such a way that the measured current does not exceed a predetermined threshold in order to avoid irritation or burning of the skin due to excessively high currents.

According to a preferred embodiment, the electric potential applied to the electrodes is varied over time. For instance, an AC field is applied between the electrodes, wherein the voltage of the AC field is between 1 V and 10 V and the frequency is between 10 kHz and 100 kHz.

As outlined above, the matrix of electrodes is preferably coated with a therapeutical agent, which may comprise one or a combination of the following substances: a growth factor, e.g. an epidermal growth factor, polymer or lipid micells and/or vesicles suitable for drug delivery, corticosteroid-based drugs, anti-inflammatory proteins.

The device and method according to the present invention provide several advantages over the prior art. Since the current applied during the therapy or stimulation is measured and controlled, skin irritation or even burning can be avoided. A considerable lateral movement of cells may be achieved due to the applied currents. The wound and its process of healing can be monitored during the therapy or stimulation. Accordingly, at every stage of healing the patch of electrodes may be controlled in response to the actual status of the wound. Due to the matrix technology any type of field or waveform can be applied allowing for an enormous versatility of the inventive device.

With the inventive device and method there are many possibilities for influencing the way a wound heals. In many cases the focus will be purely on accelerating healing of chronic wounds, but there may also be driving schemes which are suitable to reduce or avoid scarring. This could extend the application of the device from the area of chronic wounds to scar avoidance post-surgery, for example in cosmetic surgery.

These and other aspects of the invention will be apparent from and elucidated with reference to the embodiment(s) described hereinafter.

### BRIEF DESCRIPTION OF THE DRAWINGS

Figure 1 shows a flexible array of electrodes as now used in displays.
Figure 2 schematically illustrates how a device according to the present invention is used.
Figure 3 illustrates an applied voltage gradient towards the wound.
Figure 4 shows a traveling wave driving towards the wound.
Figure 5 shows a low field driving towards the wound.

### DETAILED DESCRIPTION OF EMBODIMENTS

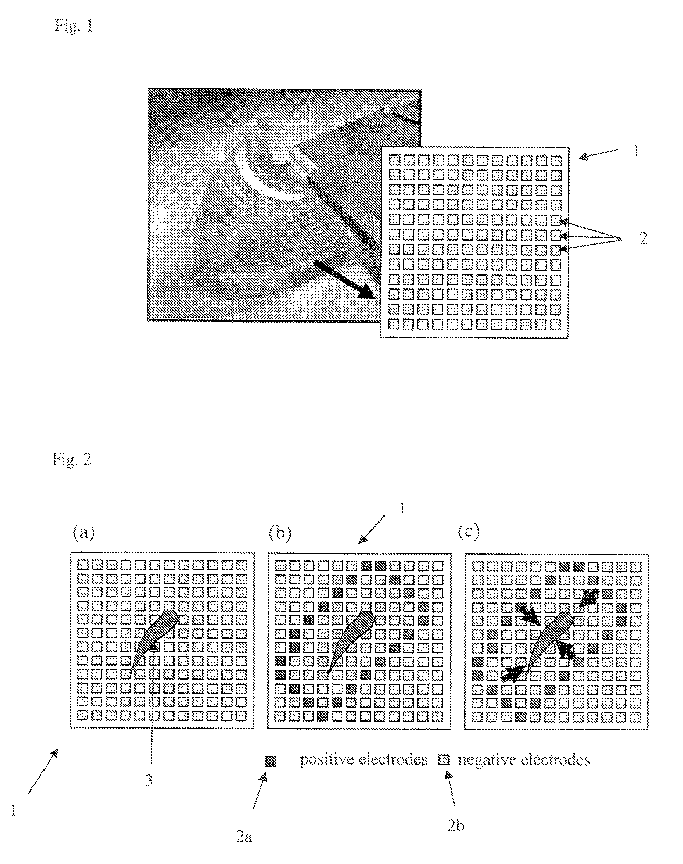

Figure 1 shows a flexible array 1 of electrodes 2 as now used in displays. Such flexible arrays 1 can be made from large area electronics technology on a polyimide release layer, which is often referred to as EPLAR. Similar technologies may be applied for the device according to the present invention.

Figure 2 schematically illustrates how a device according to a preferred embodiment of the present invention is used. A patch or matrix 1 of electrodes is placed on top of the wound 3 (see Figure 2(a)). The electrodes 2 are then scanned and the impedance between each electrode and its neighbouring electrodes is measured. Since wounded skin will have a different conductivity compared to healthy skin, the presence and location of a wound 3 and therefore its perimeter can be determined. As indicated in Figure 2(b), the electrodes 2b around and on top of the wound are made, e.g., negative. Other electrodes 2a, further away from the wound, are made positive, or vice versa. There are several options for this, the most simple is to charge the electrodes 2a at a fixed distance from the perimeter of the wound 3 positive and to charge the same number positively as were made negative.

The current or voltage waveform is applied for a fixed period of time. The resulting current from the positive electrodes 2a to the negative electrodes 2b is indicated with arrows in Figure 2(c). The amount of current that can be supplied via each segmented electrode is limited to avoid burning. If too large a current is supplied then the current is redistributed to neighbouring electrodes.

The step of scanning the electrodes is repeated and the size and conductivity of the wound is compared to the previous measurement(s). The current or waveform can then be adjusted accordingly to enhance healing. Alternatively the monitoring is not done via measuring the drawn current but by measuring the impedance of the skin under each electrode. This is done at either a pre-determined frequencies or by performing a frequency sweep. From the impedance data the state of the wound (both size and stage of healing) can be extracted and compared to previous measurements.

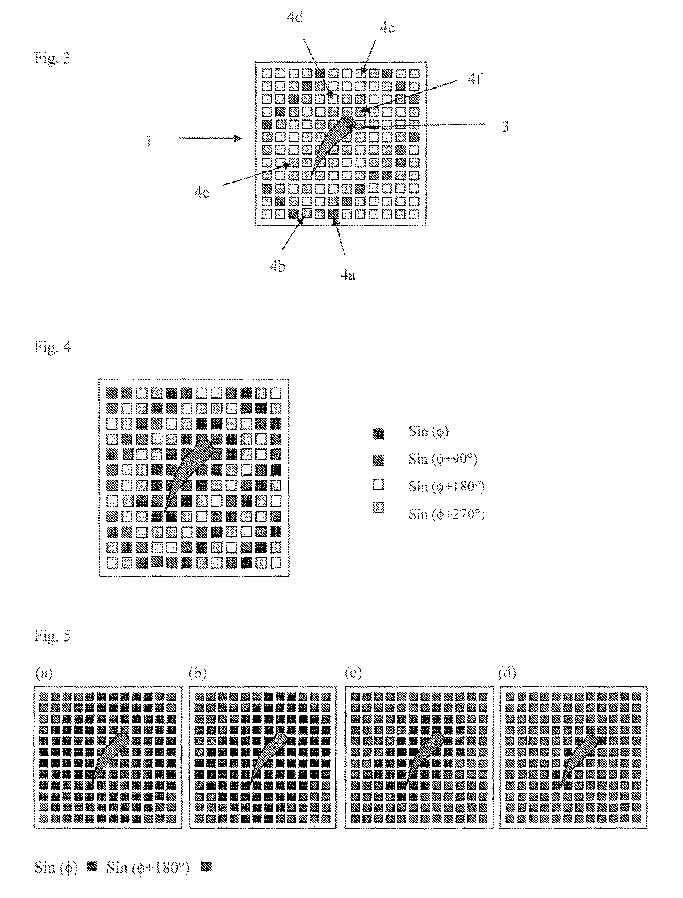

Alternatively to the embodiment shown in Figure 2, it may be preferred not to just create one ring of positive electrodes but to create a gradient in voltage from the electrodes 2a in the most outer regions of the patch towards the electrodes 2b situated near or on top of the wound. For example, several rings 4a to 4f may be set on different potentials with the potential gradually increasing or decreasing from the centre of the wound to the periphery of the patch or matrix. This is illustrated in Figure 3.

As mentioned above, such an array patch allows the generation of time-variable in-plane electrical fields. This creates many new methods for driving the electrodes which were not possible in the case of mono-electrode patches. For example, it has been shown that applying a short DC current pulse is effective for healing. With such an array patch it is possible to create a pulse on an electrode and to allow this pulse to travel towards the wound.

Alternatively it is also possible to apply AC electric fields instead of DC fields. The physical effect of dielectrophoresis could then be used to stimulate the movement of cells towards the wound. The voltage applied is typically a few volts and in the frequency range of 10-100 kHz. For example a so called travelling wave driving waveform can be seen in Figure 4 where different phases of sine wave voltage are applied to electrodes which surround the wound. This type of driving is however not the most effective if the cells are in a saline environment as is the case on a wound. Thus, one may alternatively use a low field driving scheme, wherein an area of low electrical field is created and moved in the direction of the wound. This is illustrated schematically in Figures 5(a) to 5(d).

In the embodiments described above, the migration of cells to the wound has been a purely electrically driven process. It is, however, also possible to envisage a process which is indirectly driven by electrical field but is of chemical nature. Cell migration in the body is usually driven chemically and is referred to as chemotaxis. The cells at inflammation sites excrete various proteins which include cellular growth and differentiation factors. Specific cell types (immune cells, or stem cells, for example) respond to these chemically excreted signals and migrate towards the source. If a gradient in growth factor could be created towards the wound then this would also promote cell migration.

Charged molecules such as drugs or proteins can be given to a patient transdermally via a process called iontophoresis. This involves applying an electrical field through the skin and "pulling" the charged drug particles into the patient. This is becoming a popular method for sustained drug delivery.

According to a preferred embodiment, the electrodes are coated with a growth factor such as EGF (epidermal growth factor) and a gradient in voltage towards the wound is applied (such as in Figure 3). This will "inject" the growth factor into the skin with a gradient which is determined by the voltages applied to the segmented electrodes. The growth factor gradient is therefore in the direction of the wound and should therefore promote cell migration and accelerated healing.

In addition there has been a lot of developments recently in the area of drug delivery using polymer and lipid micells and vesicles. The composition of these vesicles can be tailored to encase drugs (or growth factors) while the surface of the vesicle can be tailored to a specific charge. Hence such a "drug delivery" vesicle can be designed such that it responds to the electrical fields in a distinct manner from the natural environment (i.e. inflammatory inducing cells). This can ensure the movement and localization of only said drug delivery vehicle, whilst leaving the surrounding tissue unperturbed.

The stimulated migration of cells towards damaged tissue can have great benefits with respect to healing and tissue repair. Like-wise, persisting inflammations (i.e. non-open wounds, with swelling, necrosis and inflammation) are often treated with inflammation suppression drugs. Such drugs act to suppress the localization and/or activation of immuno-cell at the sites of inflammation. Nonetheless, these drugs are not recommended for long term use, due to side effects and cellular adaptations. Much like cells can be stimulated electrically to migrate towards a source, one can also "repel" cells electrically and chemically. Similar to the embodiment described above, the electrically active patch or matrix can be applied over an inflamed area and the electric fields tuned to repulse inflammatory cells. This may provide a superior option to corticosteroid-based drug therapies, since the negative effects of long term use would be ablated.

In addition, as described above, the electrically active patch can also be used to create a locally defined gradient of chemo-repellant either via a charged drug, or anti-inflammatory protein, or via a complex drug delivery vehicle.

In addition temperature sensors can be integrated into the electrically active patch so that the temperature of the wound can be measured. This is advantageous for detecting the presence of an infection as the skin temperature typically increases from 32C to 37C when a wound becomes infected. These sensors can also be formed in an array to allow a temperature map of the wound to be created. Most preferably the electrode geometry and material are chosen so that the electrode pad functions as both a resistive temperature sensor, a means for measuring impedance, and also for applying the voltage or current for healing.

In addition heaters can be integrated into the active patch so that the temperature profile over the wound can be controlled to influence the healing process.

Alternatively the mositure of the wound can also be measured via theelectrically active patch and this measurement used to monitor the healing process.

Alternatively the active patch can use high frequency radiation, such as THz radiation, generated locally on the patch to monitor the healing process.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. Device for treatment of a skin wound (3) comprising a flexible substrate adapted for attaching to the skin wound and the surrounding skin, a matrix (1) of electrodes (2), means for determining the presence of the wound (3) and/or its perimeter and means for applying a voltage between the wound and its surrounding.

2. Device as claimed in claim 1, wherein the means for determining the presence of the wound (3) comprise one or more sensors for detecting the real-time state of the wound (3).

3. Device according to claim 1 or 2, wherein the means for applying a voltage is adapted to control the matrix (1) of electrodes in such a way that the electrodes (2b) at and/or close to the wound (3) are charged opposite with respect to the electrodes (2a) surrounding the wound (3) or its perimeter at a predetermined distance.

4. Device according to claim 1 or 2, wherein the means for applying a voltage is adapted to control the matrix (1) of electrodes in such a way that the electric potential gradually increases or decreases from the electrodes (2b) at and/or close to the wound (3) towards the electrodes (2a) surrounding the wound or its perimeter at a predetermined distance.

5. Device according to claim 1 or 2, further comprising means for measuring the electric current caused by the applied voltage.

6. Device according to claim 5, wherein the means for applying a voltage is adapted to control the matrix (1) of electrodes (2) in such a way that the measured current does not exceed a predetermined threshold.

7. Device according to claim 1 or 2, wherein the means for applying a voltage is adapted to vary the applied voltage over time.

8. Device according to claim 1 or 2, wherein the matrix (1) of electrodes (2) is coated with a therapeutical agent.

9. Device according to claim 8, wherein the therapeutical agent comprises one or a combination of: a growth factor, e.g. an epidermal growth factor, polymer or lipid micells and/or vesicles suitable for drug delivery, corticosteroid-based drugs, anti-inflammatory proteins.

10. Device according to claim 1, wherein the device comprises large area electronics on a polyimide release layer.

11. Device according to claim 3, wherein a counter electrode is present around the electrodes (2b) at and/or close to the wound (3)

12. Device according to claim 1, wherein one or more electrodes (2) are configured to function as heater and/ or sensor.

13. Device according to claim 1, further comprising a matrix of heaters and/ or sensors.

14. Device according to claim 3, further comprising means for measuring the ac impedance of the skin or wound under an electrode.

15. Device as claimed in any of the proceeding claims, wherein one or more electrodes (2) are arranged to measure moisture of wound.

16. Method for electrical treatment of a skin wound comprising the following steps:
- placing a matrix of electrodes attached to a flexible substrate on top of the skin wound;
- determining the presence of the wound and/or its perimeter; and
- applying a different potential to the electrodes at and/or close to the wound and to the electrodes surrounding the wound at a distance.

17. Method according to claim 16, wherein steps b) and c) are repeated in predetermined time intervals.

18. Method according to claim 16, wherein the electric potential gradually increases or decreases from the electrodes at and/or close to the wound towards the electrodes surrounding the wound or its perimeter at a predetermined distance.

19. Method according to claim 16, further comprising the step of measuring the current between the electrodes.

20. Method according to claim 19, wherein the potential applied to the electrodes is controlled in such a way that the measured current does not exceed a predetermined threshold.

21. Method according to claim 16, wherein the presence of the wound and/or its perimeter is determined by measuring the impedance between each electrode of the matrix and its neighboring electrodes.

22. Method according to claim 16, wherein the electric potential applied to the electrodes is varied over time.

23. Method according to claim 22, wherein an AC field is applied between the electrodes.

24. Method according to claim 23, wherein the voltage of the AC field is between 1 and 10 V and the frequency is between 10 and 100 kHz.

25. Method according to claim 16, wherein the matrix of electrodes is coated with a therapeutical agent.

26. Method according to claim 25, wherein the therapeutical agent comprises one or a combination of: a growth factor, e.g. an epidermal growth factor, polymer or lipid micells and/or vesicles suitable for drug delivery, corticosteroid-based drugs, anti-inflammatory
